# EUROPEAN PATENT APPLICATION

(11) **EP 0 564 941 A1**
(43) Date of publication of application: **13.10.1993**
(21) Application number: 93105140.3
(22) Date of filing: 29.03.1993
(51) Int. Cl.: A61B 17/58

(54) **Intramedullary pin for dynamic osteosynthesis with a self-locking distal end for fractures of the humeral metadiaphyseal region**

(30) Priority: 06.04.1992 IT BO920120
(71) Applicant: Zoli, Andrea, I-48010 Cotignola (Ravenna) (IT)
(72) Inventor: Zoli, Andrea, I-48010 Cotignola (Ravenna) (IT)
(74) Representative: Modiano, Guido, Dr.-Ing.

(57) **Abstract**

Intramedullary pin for dynamic osteosynthesis with a self-locking distal end for fractures of the humeral metadiaphyseal region, including a proximal stub (2) whose upper part is inclined and crossed by a hole (4) along which the stem of a screw (5) can be screwed; the screw can be rigidly associated with the opposite cortex of the bone; the ends of at least two curved and resilient stems (9) are axially rigidly coupled to the stub; the stems are provided, at their free ends, with elements (10-12) for temporary mutual coupling which are suitable to keep the tips of the stems mutually adjacent during the insertion of the pin in the medullary canal through one of the two humeral metadiaphyses and to release the tips when the pin is in correct position to allow the elastic spread of the stems, whose tips anchor in the intramedullary spongy bone, sometimes to the extent of pressing against the internal walls of the bone, rigidly coupling the synthesis element and the bone.

## Description

The present invention relates to an intramedullary pin for dynamic osteosynthesis with a self-locking distal end for fractures of the humeral metadiaphyseal region.

In order to reduce fractures affecting the metadiaphysis of the humerus, it is known to use an intramedullary pin or pins or plates which are anchored to the intact cortex of the long bone by means of screws or mechanical-expansion systems. In order to fix intramedullary pins to the humeral diaphysis it is known, in these operations, to work by mechanical expansion or with transverse screws which are screwed into the cortices: this fixing by means of screws entails some drawbacks which are due, among other things, to the fact that additional surgery time is required and that it is usually necessary to resort to radiological means for rather prolonged periods in order to determine the fixing position of the screws or pins, with consequent exposure of the surgeon, of the assistants and of the operating-theater staff to harmful ionizing radiation.

In addition, when the pin must be removed at the end of the treatment, it is necessary to intervene not only in the region of the bone from which the pin must be extracted but also distally, where the fixing screws must be removed.

An intramedullary pin with a self-locking end for metadiaphyseal fractures of long bones of the leg was devised and patented some time ago; said intramedullary pin has yielded excellent results as regards the ease and rapidity of anchoring of the self-locking distal end: in view of this, it has been thought to provide an intramedullary pin for dynamic osteosynthesis of fractures of the humeral metadiaphysis.

An aim of the present invention is to provide a pin which does not require external intervention for the distal fixing of the pin to the bone, which allows elastic and dynamic osteosynthesis, which can be installed very rapidly requiring a very low exposure to radiation, which does not require drilling and preliminary preparation of the medullary canal, and which can be even more easily removed once the fracture has set.

Another aim the present invention is to provide a pin which can be installed with an extremely simple set of instruments, and which has a low cost and adapts to various dimensional conditions of the bone in which it is installed, so that a very small number of sizes is sufficient to meet any requirement.

Another object of the present invention is to achieve the above aim with a structure which is simple, relatively easy to produce in practice, safe in use and effective in operation as well as relatively modest in cost.

This aim and these objects are all achieved by the present intramedullary pin for dynamic osteosynthesis with a self-locking distal end for fractures of the humeral metadiaphyseal region, characterized in that it comprises a proximal stub whose upper part is inclined and crossed by a hole along which the stem of a screw can be screwed; said screw being rigidly associable with the opposite cortex of the bone; the ends of at least two curved and resilient stems being axially rigidly coupled to said stub; said stems being provided, at their free ends, with means for temporary mutual coupling; said means being suitable to keep the tips of the stems mutually adjacent during the insertion of the pin in the medullary canal through one of the two humeral metadiaphyses and to release the tips when the pin is in correct position to allow the elastic spread of the stems, whose tips anchor in the intramedullary spongy bone, sometimes to the extent of pressing against the internal walls of the bone, rigidly coupling the synthesis means and the bone.

Further particularities and advantages of the present invention will become apparent and evident from the following detailed description of a preferred but not exclusive embodiment of an intramedullary pin according to the invention, illustrated only by way of non-limitative example in the accompanying drawings, wherein:
figure 1 is a perspective view of an intramedullary pin according to the invention, with its stems gathered;
figure 2 is a perspective view of an intramedullary pin according to the invention, after the release of the stems;
figure 3 is an enlarged partially cutout perspective side view of the pin;
figure 4 is an enlarged partially cutout perspective side view of the pin with a sort of actuation handle installed;
figures 5 and 6 are schematic side views of two installations of the pin according to the present invention in fractures of the lower part and of the upper part of the humerus respectively.

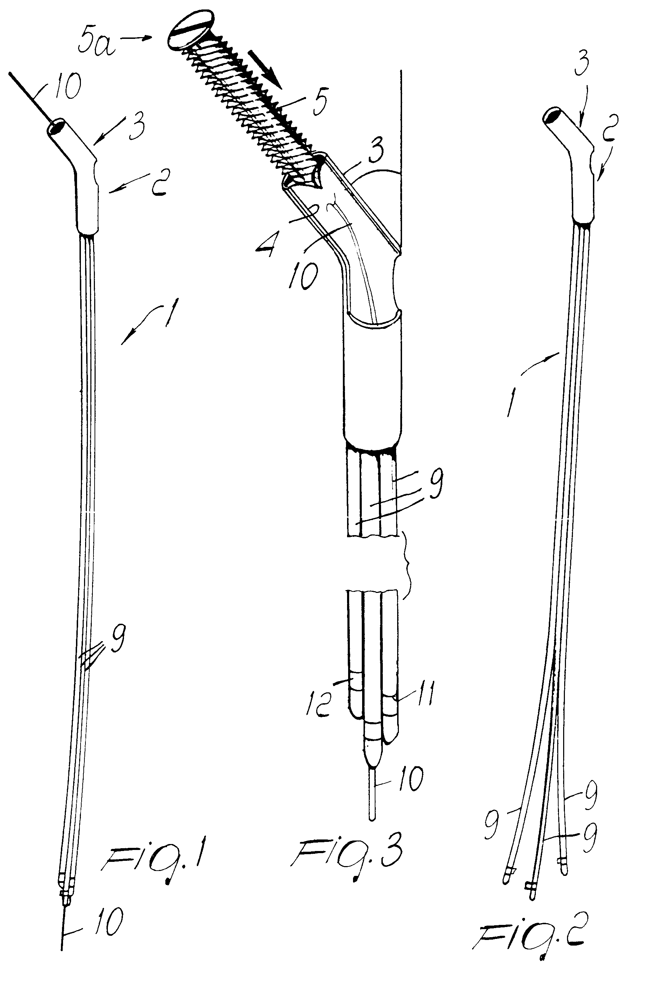

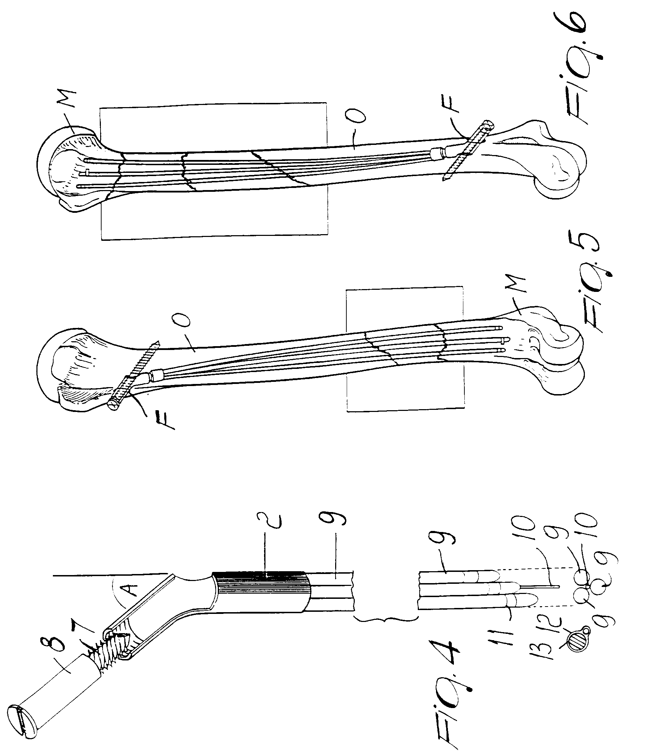

With particular reference to the above figures, the reference numeral 1 generally designates the intramedullary pin according to the present invention for fractures of the metadiaphyseal region of the humerus O.

The pin 1 comprises a proximal stub 2 whose upper part 3 is inclined and crossed by a threaded hole 4 along which the stem of a self-threading screw 5 for coupling to the cortex can be screwed.

The stub 2 forms, together with the upper part 3, an angle A comprised between 40 and 50 degrees so as to assume a more correct anatomical configuration, improve load distribution and avoid fractures of the cortex during the insertion of the pin.

The threaded end 7 of a stem 8 can be screwed in the threaded hole 4 of the stub; said stem 8 forms a sort of handle or grip instrument which facilitates the operations for inserting and extracting the pin in and from the bone.

At its other end, at least two resilient curved stems 9, three in the particular illustrated case, are rigidly coupled to the stub 2 (advantageously, the stems are made of a biocompatible steel such as for example AISI 316 L); the stems can advantageously have a circular cross-section and rounded ends.

Conveniently, the base of the stub is shaped like a frustum-shaped cone which converges slightly toward the couplings of the stems.

Means for temporarily coupling the stems in a bundle are provided and can consist, for example, of a metallic wire 10 at one end whereof a ring for manual grip is rigidly coupled; proximate to their ends, the stems 9 are provided with respective annular grooves 11 in which oval rings 12 are arranged; said rings are compressed so as to rigidly couple to the grooves, substantially assuming the shape of the numeral 8 so as to define respective holes 13 in which the metallic wire 10 is insertable; conveniently, the wire is made to pass longitudinally inside the stub 2.

The operation of the pin according to the present invention is as follows: the pin is inserted in the medullary canal from one of the ends of the humerus, on one side of which an insertion hole F is produced; using a radiographic investigation means, the pin is pushed until it reaches the opposite metadiaphysis of the humerus; at this point, the wire 10 is pulled and releases the stems, allowing them to spread out; as the insertion of the pin proceeds, the stems, by spreading out, press the bone of the metadiaphysis M from the inside and anchor themselves to it.

At this point it is possible to drill the cortical bone on the side opposite to the one drilled earlier, passing through the hole 4 of the upper part of the stub.

By acting on the notch 5a, it is then possible to screw the screw 5 at 4; the self-threading end of said screw screws itself into the bone cortex which is opposite to the one where insertion occurred.

The fact is stressed that the self-locking end fixes itself to the distal humerus by virtue of an automatic elastic mechanism, without having to intervene from the outside on this region; furthermore, since the various stems arrange themselves on different planes, torsional movements of the stub with respect to the bone are prevented.

It is stressed that a small number of sizes of the pin according to the invention are sufficient to be able to operate on any kind of patient and fracture and that no preliminary operations for drilling or preparing the medullary canal are necessary.

It has thus been observed that the invention achieves the intended aim and objects.

The invention thus conceived is susceptible to numerous modifications and variations, all of which are within the scope of the inventive concept.

All the details may furthermore be replaced with other technically equivalent ones.

In practice, the materials employed, as well as the shapes and dimensions, may be any according to the requirements, without thereby abandoning the protective scope of the following claims.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. Intramedullary pin (1) for dynamic osteosynthesis with a self-locking distal end for fractures of the humeral metadiaphyseal region, characterized in that it comprises a proximal stub (2) whose upper part (3) is inclined and crossed by a hole (4) along which the stem of a screw (5) can be screwed; said screw being rigidly associable with the opposite cortex of the bone; the ends of at least two curved and resilient stems (9) being axially rigidly coupled to said stub; said stems being provided, at their free ends, with means (10-12) for temporary mutual coupling; said means being suitable to keep the tips of the stems mutually adjacent during the insertion of the pin in the medullary canal through one of the two humeral metadiaphyses and to release the tips when the pin is in correct position to allow the elastic spread of the stems, whose tips anchor in the intramedullary spongy bone, sometimes to the extent of pressing against the internal walls of the bone, rigidly coupling the synthesis means and the bone.

2. Pin according to claim 1, characterized in that said hole (4) has an axis which is arranged on a plane which is substantially radial with respect to the axis of the stub (2) and forms therewith an angle comprised between 40 and 50 degrees.

3. Pin according to claim 1, characterized in that said means for the temporary mutual coupling of the stems comprise respective rings (12) which are compressed so as to rigidly couple in corresponding peripheral grooves (11) of the ends of the stems (9) so as to define respective holes (13) in which a retention wire (10) is inserted, said wire extending along the stems and being removable from the end of the stub to release the stems.

4. Pin according to claim 1, characterized in that the upper inclined part (3) of the stub has an axial threaded hole (4) for the bone anchoring screw (5) and for screwing a sort of grip handle, said handle being suitable to facilitate the operations for inserting and extracting the pin in and from the medullary canal.

5. Pin according to claim 1, characterized in that said stub has a base shaped like a slightly converging frustum-shaped cone.

6. Pin according to claim 3, characterized in that said stub is axially perforated for the passage of said metallic wire.
